# EUROPÄISCHE PATENTANMELDUNG

(11) **EP 2 221 053 A1**
(43) Veröffentlichungstag der Anmeldung: **25.08.2010**
(21) Anmeldenummer: 09002395.3
(22) Anmeldetag: 20.02.2009
(51) Int. Cl.: A61K 31/415, A61K 31/4196, A61K 31/4245, A61K 31/426, A61K 31/433, A61K 31/444, A61K 31/505, A61K 31/513, A61K 31/52, A61K 31/53, A61K 31/165, A61K 31/568, A61K 31/5685, A61K 45/06, A61P 35/00

(54) **Pharmazeutische Zusammensetzung enthaltend Hemmstoffe der Proteinmethyltransferase I und deren Verwendung zur Behandlung von Tumorerkrankungen**

(71) Anmelder: Albert-Ludwigs-Universität Freiburg, 79085 Freiburg (DE); Martin-Luther-Universität Halle-Wittenberg, 06108 Halle/Saale (DE)
(72) Erfinder: Jung, Dr. Manfred, 71994 Gundelfingen (DE); Schüle, Dr. Roland, 79367 Weisweil (DE); Metzger, Dr. Eric, 68600 Neuf-Brisach (FR); Spannhoff, Astrid, 49536 Lienen (DE); Liebscher, Dr. Jürgen, 12524 Berlin (DE); Pätzel, Dr. Michael, 15370 Fredesdorf (DE); Sippl, Dr. Wolfgang, 06120 Halle (DE)
(74) Vertreter: Keller, Günter

(57) **Zusammenfassung**

Offenbart werden Inhibitoren der PMRT-Typ I-Methyltransferase und deren Verwendung in pharmazeutischen Zusammensetzungen, die eine Verbindung der allgemeinen Formel I 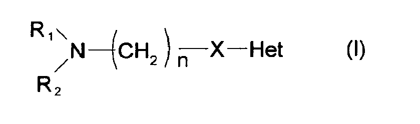 enthalten, worin die Reste R₁ und R₂ gleich oder unterschiedlich sind und ausgewählt sind aus den Bedeutungen Wasserstoff, gerad- oder verzweigtkettiges C₁₋₅-Alkyl, oder 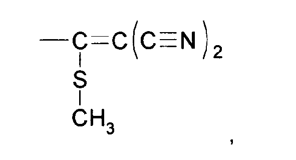 n eine ganze Zahl zwischen 3 und 5 ist,

X ein Heteroatom ist, ausgewählt aus O, S, N-R₁ oder die Bedeutung -CH₂- hat und der Rest Het ein 5-, 6- oder 7-gliedriger aromatischer oder nicht aromatischer Ring ist, der 1 bis 5 Heteroatome, ausgewählt aus N, S und O ist, wobei der Ring gegebenenfalls substituiert sein kann und ein substituierter oder nicht substituierter polycyclischer Ring sein kann sowie dessen pharmazeutisch annehmbare Salze. Die Zusammensetzungen dienen der Verwendung als Arzneimittel gegen Tumorerkrankungen.

## Beschreibung

Die vorliegende Erfindung betrifft Hemmstoffe der Proteinmethyltransferase-1 (PRMT-I) und deren Verwendung in pharmazeutischen Zusammensetzungen, insbesondere für die Therapie von Tumorerkrankungen.

Proteinmethyltransferasen und insbesondere die Protein-Arginin-Methyltransferase katalysieren die posttranslationale Methylierung von Argininresten in Proteinen, die zu der Mono- und Dimethylierung von Arginin an der Guanidinogruppe führen. Interessante Substrate sind die Histone oder heterologe nucleare Ribonucleoproteine (hnRNPs). Bekannt sind die beiden PRMT-Typen I und II, die beide Arginin über Monomethylarginin zu Dimethylarginin methylieren. Die beiden Typen unterscheiden sich darin, dass der Typ I ein asymmetrisches Dimethylarginin mit zwei Methylgruppen an einem Stickstoffatom erzeugt, wohingegen bei PRMT vom Typ II ein symmetrisches Dimethylarginin entsteht, wobei jedes Stickstoffatom der Guanidinogruppe je eine Methylgruppe aufweist. Das Dimethylarginin, das durch PRMT-II erzeugt wird, weist also an je einem Amino-Rest des Arginins eine Methylgruppe auf, wohingegen bei der Methylierung mit PRMT-I ein Arginin entsteht, bei dem beide Methylgruppen an eine Aminogruppe des Arginins gebunden sind.

Gegenstand der vorliegenden Erfindung sind Verbindungen, die die PRMT-I inhibieren sowie deren Verwendung in pharmazeutischen Zusammensetzungen, die insbesondere bei der Therapie von Krebserkrankungen eingesetzt werden können.

Histon modifizierende Enzyme sind wichtige Schlüsselproteine in der epigenetischen Regulation eukaryontischer Zellen. Durch posttranslationale Modifikationen der N-terminalen Aminosäuren durch solche Enzyme werden Signale gesetzt, welche den Zugriff von Transkriptionsfaktoren auf die Erbsubstanz (DNS) steuern. Diese Modifikationen wirken nicht isoliert, sondern ermöglichen in ihrer gegenseitigen Beeinflussung eine gezielte Regulation der betreffenden Gene. Daher wird die Existenz eines sich aus diesem Zusammenspiel ergebenden sogenannten Histon-Codes postuliert, der bei Erkrankungen fehlcodiert ist und durch Arzneistoffe korrigiert werden soll. Die Funktion von Histonacetyltransferasen und Histondesacetylasen (HDACs) bei zentralen zellulären Vorgängen ist detailliert beschrieben und ihre Rolle bei der Entstehung maligner Erkrankungen zählt zu den am besten untersuchten Gebieten im Bereich der Epigenetik.

HDAC-Inhibitoren befinden sich z.Z. in der klinischen Prüfung oder haben bereits eine Zulassung erhalten. Für Histonmethyltransferasen exisitieren weniger Erkenntnisse zur Beteiligung am Krankheitsgeschehen und es sind bislang auch nur wenige Hemmstoffe bekannt. Die Methylierung von Histonen stellt eine komplexere Modifikation als die Acetylierung dar. Sie kann sowohl an Argininen als auch Lysinen auftreten, wobei je nach Modifikationsstelle die Transkription gesteigert oder vermindert wird. Zur Komplexität trägt auch die Tatsache bei, dass die Methylierung in unterschiedlichen Graden auftreten kann. Lysin kann mono-, di-, oder trimethyliert werden, beim Arginin verläuft die Methylierung zum Mono- oder Dimethylarginin. Die Dimethylierung kann zudem symmetrisch durch PRMT-II (zwei Methylgruppen an zwei verschiedenen Stickstoffatomen der Guanidingruppe des Arginins) oder asymmetrisch durch PRMT-I (beide Methylgruppen am gleichen Stickstoffatom) verlaufen.

Durch eine Kombination von Target basiertem virtuellem Screening und einem biochemischen Testsystem konnten Inhibitoren der Proteinmethyltransferase 1 (PRMT1) identifiziert werden.

PRMT1 katalysiert die asymmetrische Dimethylierung an Arginin 3 im Histon H4 und gehört somit zu den Typ-I-PRMTs. Das Enzym besitzt analog zu PRMT4 (CARM1) coaktivierende Eigenschaften bei der durch nukleäre Rezeptoren vermittelten transkriptionellen Regulation. PRMT1 und PRMT4/CARM 1 agieren ebenfalls als Coactivatoren für eine Vielzahl anderer Transkriptionsfaktoren, z.B. p53. D.h., die Aktivierung von Androgen- oder Estrogenrezeptoren durch die physiologischen Agonisten ist von einer Argininhypermethylierung abhängig, die durch PRMT-Hemmstoffe blockiert werden kann und daher verringert sich auch die Aktivierung des Rezeptors. Derartige Verbindungen können daher als PRMT1-Inhibitoren zur Behandlung androgen- oder estrogenabhängiger Tumoren eingesetzt werden.

Bei dem von Cheng et al. [J.Biol.Chem. (2004), 279, 23892-23899] durchgeführten Hochdurchsatz-Screening wurden die Verbindungen AMI-1 und AMI-5 als Hemmstoffe von PRMT1 entdeckt. Beide inhibieren das Enzym in-vitro im niedrig mikromolaren Bereich (IC₅₀ AMI-1: 8,8 µM, IC₅₀ AMI-5: 1,4 µM). AMI-1 wird als selektiv gegenüber der Lysinmethyltransferase SET7/9 beschrieben, AMI-5 hingegen als dualer Inhibitor. Ausgehend von diesen Farbstoff- bzw. farbstoffähnlichen Strukturen wurden Strukturwirkungsbeziehungen erstellt. Die synthetisierten Verbindungen sind jedoch zum größten Teil an PRMT1 wesentlich schwächer wirksam als die Leitstrukturen.

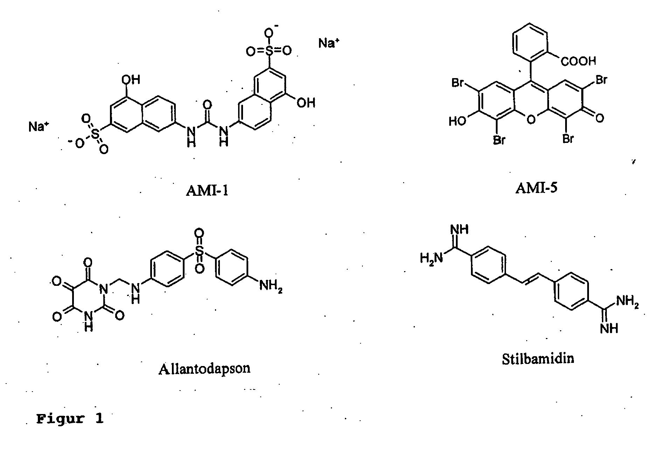

Es wurden im oben erwähnten Target-basierten Screening Allantodapson, Stilbamidin und RM-65 als Inhibitoren der PRMT (IC₅₀ Allantodapson: 1,7 µM, IC₅₀ Stilbamidin: 56,9 µM, IC₅₀ RM-65: 55,4 µM) identifiziert. Stilbamidin und RM-65 sind gegenüber SET7/9 selektiv, während Allantodapson in-vitro auch dieses Enzym hemmt (IC₅₀: 10,96µM). Durchgeführte Docking- und Kompetitionsexperimente legen den Schluss nahe, dass es sich bei Stilbamidin und Allantodapson um zum Sustrat kompetitive Verbindungen handelt, während bei RM-65 und AMI-1 von einer Bisubstratinhibition ausgegangen werden kann. Die Struktur von bekannten PMRT-Inhibitoren ist in Fig. 1 dargestellt.

Mittels eines Target basierten virtuellen Screenings der Hemmstoffe aus einer Substanzbibliothek wurden Substanzen als neue PRMT-Inhibitoren identifziert. Auch strukturverwandte Substanzen aus dieser Gruppe wurden getestet, die nicht in diesem Substanzpool enthalten waren. Die Substanzen wurden charakterisiert durch die Enzymhemmung in-vitro (an humaner PRMT1 und dem Aspergillus-Homologen RmtA), durch die zelluläre Hitvalidierung (Argininhypomethylierung in HepG2-Zellen) und durch die Messung einer funktionalen Aktivität (Unterdrückung von Androgen bzw. Estrogen vermittelter Rezeptoraktivierung im Reportergenassay). RmtA wurde für ein Vorscreening verwendet und ein IC₅₀-Wert an hPRMT1 wurde bei einer Hemmung von mehr als 50 % bei 10 µM gemessen.

Für die Enzymhemmung wurde ein Assay verwendet, der auf immobilisierten Oligopeptiden als Enzymsubstrate und den entsprechenden Enzymen beruht. Die Quantifizierung der Methylierung erfolgt über einen Antikörper gegen das Dimethylarginin und die Detektion über einen Europium gelabelten Sekundärantikörper. Die potentesten Verbindungen wurden ebenfalls in-vitro auf ihre Selektivität gegenüber SET7/9, einer Lysinmonomethyltransferase untersucht. Die Methodik der verwendeten Testverfahren ist im Detail in der Dissertation von Astrid Spannhoff, "Neue Testsysteme für Histonmethyltransferasen und Histondemethylasen", Albert-Ludwigs-Universität Freiburg, 2007 beschrieben. Der Offenbarungsgehalt dieser Arbeit wird durch Bezugnahme hier eingeschlossen.

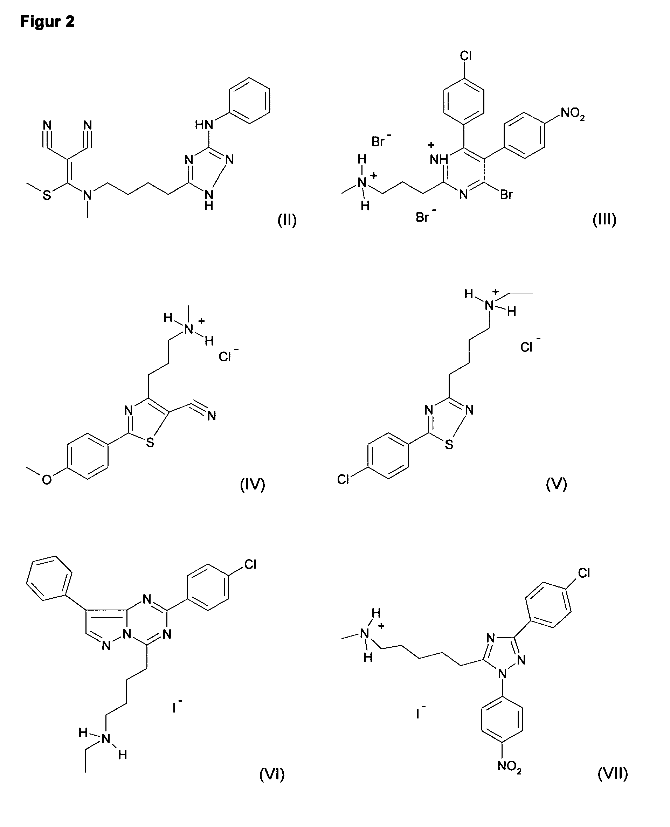

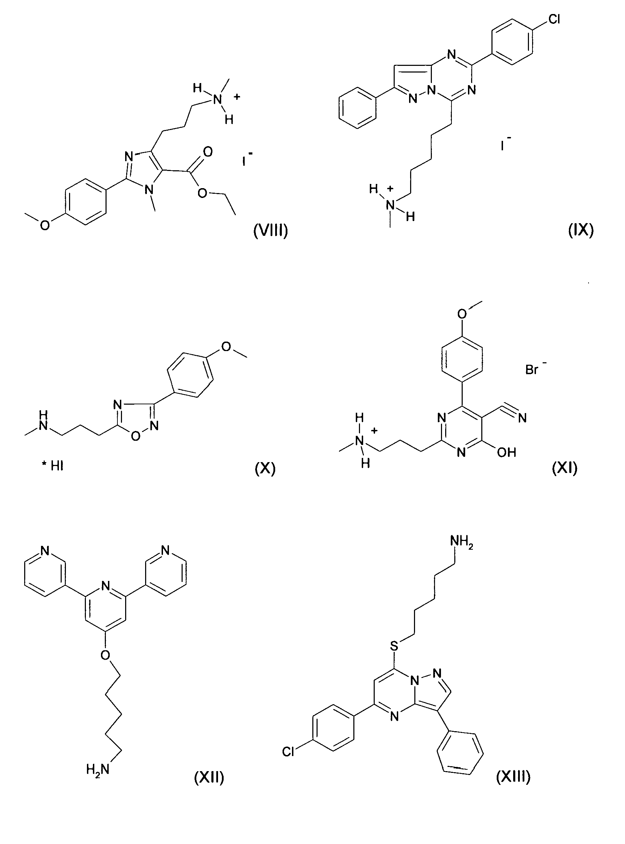

Die Strukturen der erfindungsgemäß bevorzugten Verbindungen sind in Fig. 2 dargestellt und die gemessene Argininmethyltransferasehemmung ist in Tabelle 1 zusammengefaßt. Den Strukturformeln der bevorzugten Verbindungen wurde eine römische Zahl zugeordnet, die in der Beschreibung anstelle der jeweiligen Verbindung verwendet wurde.

**Tabelle 1. Argininmethyltransferasebemmung**

| **Verbindung der Formel** | **Hemmung RmtA** (%Inh. bei 10 µM) | **Hemmung PRMT1** (% Inh bei 10 µM oder IC₅₀ + SD, µM) |
|---|---|---|
| II | 31,7 % | |
| III | 30,8 % | |
| IV | 55,0 % | |
| V | 38,7 % | |
| VI | 83,4 % | 4,18 ± 3,5 µM |
| VII | 46,4 % | 11, 7 % |
| VIII | 35,0 % | |
| IX | 43,64 % | 10,38 + 2,0 µM |
| X | 23,1 % | |
| XI | 23,0 % | |
| XII | 53,7 % | 3,10 + 0,2 pM |
| XIII | 22,8 % | 9,32 + 2,68 µM |

Gegenstand der vorliegenden Erfindung sind pharmazeutische Zusammensetzungen, die wenigstens einen Inhibitor der PRMT-Typ 1-Methyltransferase aufweisen. Bei den erfindungsgemäßen Inhibitoren ist eine strukturelle Gemeinsamkeit erkennbar, die in Formel (I) 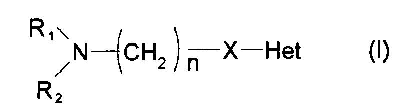 dargestellt ist. An dem Stickstoffatom sind zwei Reste R₁ und R₂ vorhanden, die gleich oder unterschiedlich sein können und die Bedeutung Wasserstoff, gerad- oder verzweigtkettiger C₁₋₅-Alkylrest oder die Struktur 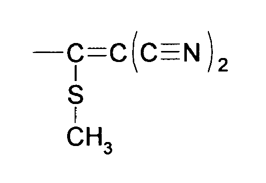 haben. Bevorzugt hat wenigstens einer der beiden Reste die Bedeutung Wasserstoff und der andere Rest hat bevorzugt die Bedeutung Methyl oder Ethyl. Das Stickstoffatom der Formel (I) kann in protonierter Form vorliegen, wenn der Inhibitor als Salz eingesetzt wird. In bevorzugter Ausführungsform werden die Inhibitoren als pharmazeutisch annehmbare Salze eingesetzt, beispielsweise als Chlorid, Hydrochlorid oder Jodid. In der Formel (I) hat n die Bedeutung einer ganzen Zahl, die zwischen 3 und 5 liegt. X kann ein Heteroatom sein, das ausgewählt ist aus der Bedeutung O (Sauerstoff), S (Schwefel) oder N-R₁, wobei dieser Rest R₁ die oben angegebene Bedeutung hat. In einer anderen Ausführungsform kann X auch die Bedeutung -CH₂- haben.

Bei dem Rest Het handelt es sich um einen 5-, 6- oder 7-gliedrigen aromatischen oder nicht aromatischen Ring, gegebenenfalls mit zusätzlich annelliertem 5- oder 6-Ring, der 1 bis 5 Heteroatome, ausgewählt aus N, S und O aufweisen kann, wobei dieser Ring gegebenenfalls substituiert sein kann. Diejenige Bindung über die der Rest Het an die Strukturen der Formel (I) gebunden ist, ist durch ein Symbol " 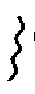 " gekennzeichnet.

In bevorzugter Ausführungsform handelt es sich bei dem Rest Het um einen 5- oder 6-Ring der 1-3 Stickstoffatome und 1-2 weitere Heteroatome, ausgewählt aus Schwefel und Sauerstoff enthält. Das Gesamtringsystem ist maximal ungesättigt.

In bevorzugter Ausführungsform ist der Rest Het ein gegebenenfalls substituierter heterocyclischer Ring mit der Formel IA 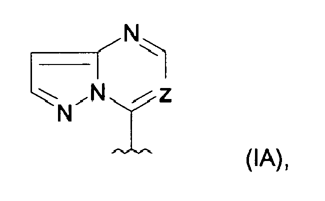 in dem Z die Bedeutung CH oder N hat.

Der Substituent Het mit der oben wiedergegebenen Formel IA ist bevorzugt mit 1 oder 2 Substituenten substituiert, wobei die Substituenten bevorzugt ausgewählt werden aus gegebenenfalls substituierten Phenylresten.

Es kann sich bei Het also um einen substituierten oder nicht substituierten polycyclischen Ring handeln, wobei die bevorzugten Strukturen nachfolgend wiedergegeben sind:

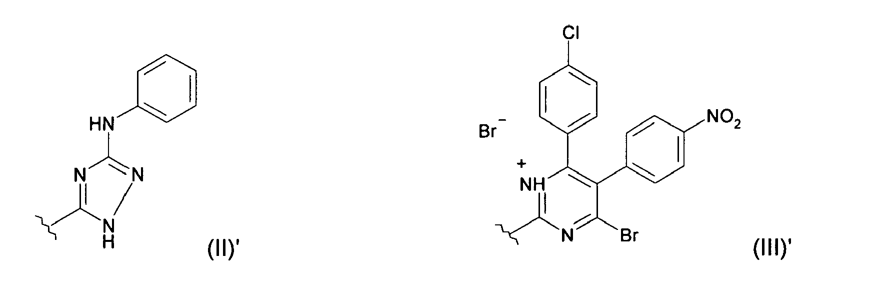

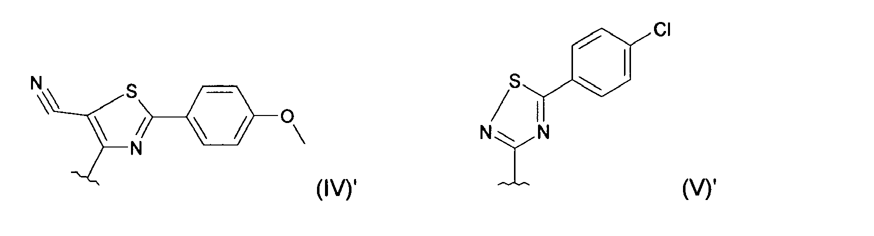

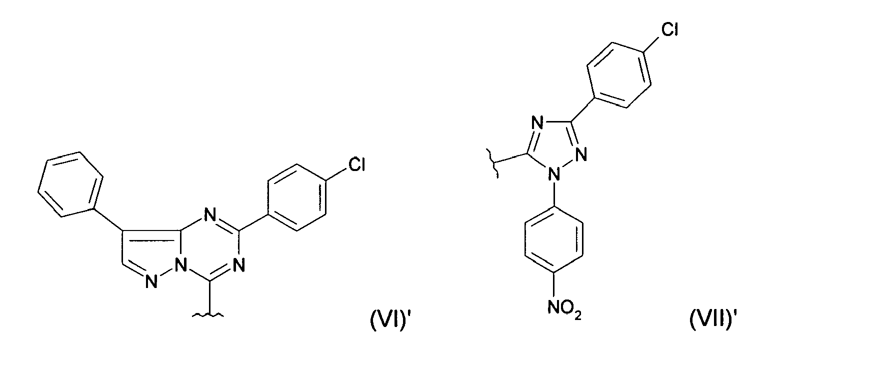

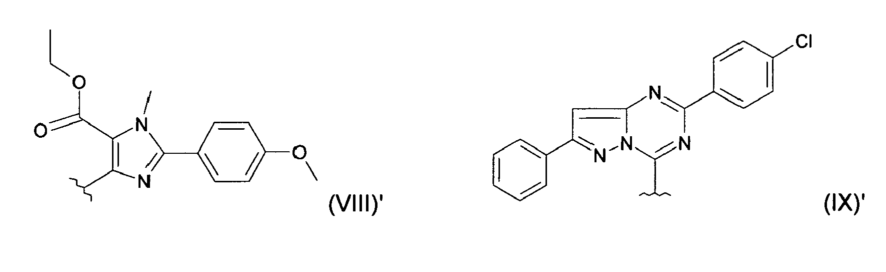

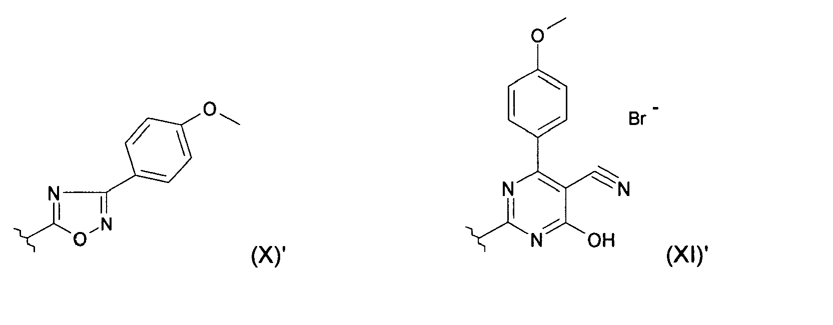

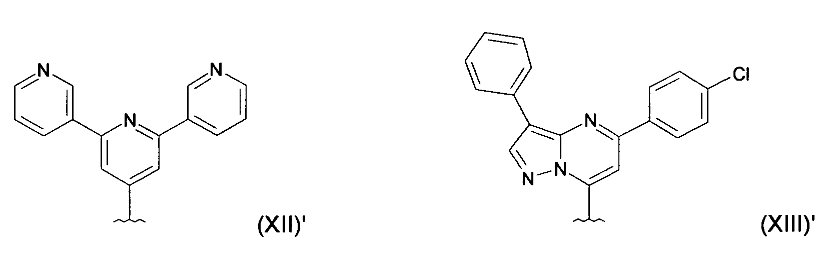

In einer besonders bevorzugten Ausführungsform wird in den pharmazeutischen Zusammensetzungen wenigstens eine der in Fig. 2 dargestellten Verbindungen eingesetzt.

Die erfindungsgemäßen Zusammensetzungen werden als Arzneimittel eingesetzt und dienen bevorzugt der Verwendung als Antikrebsmittel. In bevorzugter Ausführungsform können die pharmazeutischen Verbindungen als Mittel gegen verschiedene Krebs- oder Tumorformen eingesetzt werden, wobei sie besonders bevorzugt bei folgenden Erkrankungen eingesetzt werden können:

Blasenkarzinom, Brustkarzinom, Colonkarzinom, Nierenkarzinom, Leberkarzinom, Lungenkarzinom, kleinzelliges Lungenkarzinom, Speiseröhrenkarzinom, Gallenblasenkarzinom, Eierstockkarzinom, Pancreaskarzinom, Magenkarzinom, Zervixkarzinom, Prostatakarzinom, Karzinome der Haut, Leukämie, B-Zell-Lymphoma, T-Zell-Lymphoma, Hutchinson-Lymphoma, Non-Hutchinson-Lymphoma, Fibrosarkom, Neublastom, Melanom oder Karposi-Sarkom.

In einer weiteren bevorzugten Ausführungsform werden bei den erfindungsgemäßen pharmazeutischen Zusammensetzungen die oben beschriebenen Verbindungen in Kombination mit einer Anti-Androgen-wirksamen Verbindung eingesetzt.

Derartige Anti-Androgen-wirksame Verbindungen sind ausgewählt aus der Gruppe umfassend Androgen-Rezeptorantagonisten und 5α-Reduktasehemmer.

Als bevorzugte Beispiele für geeignete Androgen-Rezeptorantagonisten können Flutamid, Bicalutamid, Cyproteron und Cyproteronacetat genannte werden. Bevorzugte 5α-Reduktasehemmer sind Finasterid und Dutasterid.

Die erfindungsgemäßen Verbindungen können parenteral oder oral verabreicht werden und auch in topischen Formulierungen eingesetzt werden.

Es wurde ein in vitro-Testsystem für Histonmethyltransferasen verwendet, das für ein Mikrotiterplattenformat konzipiert wurde. Da sich Substrat und nicht umgesetztes Substrat weder in ihren UV- noch in ihren Fluoreszenzeigenschaften unterscheiden, wurde eine Antkörper basierte Detektionsmethode gewählt. Als primäre Antikörper wurden bevorzugt kommerziell erhältliche, in Kaninchen produzierte polyklonale Immunglobuline der Klasse G (IgG) verwendet. Diese Antikörper richten sich gegen die methylierte Form des Substrats. Wenn also ein Inhibitor die PRMT-Typ 1-Methyltransferase inhibiert, können derartige Antikörper an das Produkt der enzymatischen Reaktion nicht oder nur in reduziertem Umfang binden.

Die Bindung der primären Antikörper an das Produkt der enzymatischen Reaktion wurde mit einem sekundären Antikörper nachgewiesen. Hierbei handelt es sich bevorzugt um einen in der Ziege erstellten, gegen Kaninchen 1gG gerichteten Antikörper. Dieser Ziegenantikörper war mit einem Europium (Eu)-Label versehen, der eine Fluoreszenzmessung im zeitaufgelösten Modus ermöglichte.

Um jeweils nur den an das Produkt der enzymatischen Reaktion gebundenen Anteil an Antikörper bestimmen zu können, musste der nicht gebundene Anteil entfernt werden. Deshalb wurde das Substrat an die Wände der Mikrotiterplatte gebunden. Diese Fixierung wurde durch Verwendung des Strepavidin-Biotin-Systems bewirkt.

### Beispiel 1

### Targetvalidierung (Hypomethylierung in Zellen)

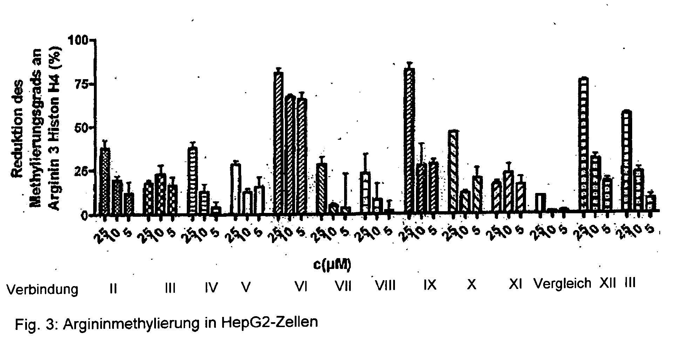

Mittels dieses ELISA-ähnlichen Testsystems wurde durch den Einsatz eines AntiDimethyl-Histone H4(Arg3)-Antikörpers ein direkter Einfluss der Testverbindungen auf den Methylierungsgrad von Arg3 im Histon H4 der Zellen nachgewiesen. Die Untersuchungen wurden an HepG2-Zellen durchgeführt, da sie sich aufgrund ihrer adhärenten Eigenschaften als geeignet erwiesen. Um Unterschiede der Testsubstanzen im Hinblick auf Zellproliferation auszugleichen, wurden die ermittelten Signale auf die tatsächlichen Zellzahlen (ausgedrückt als Proteinkonzentration) normiert. Die Ergebnisse sind in Fig. 3 dargestellt.

### Beispiel 2

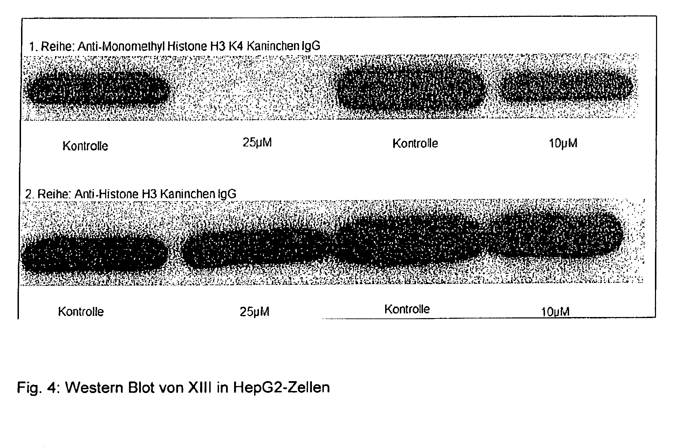

Als besonders potent (Reduktion der Methylierung an Arg3 Histon H4 > 50 % bei der Konzentration 25 µM) erwiesen sich Verbindungen der Formel XII und XIII. Die Verbindungen VI und IX zeigten zusätzlich einen stark cytotoxischen Effekt, was die Ergebnisse beeinflusste. Für die Verbindung wurde die zelluläre Hypomethylierung mittels Western Blot bestätigt (Reduzierung der Methylierung bei 25 µM um 99,98%; bei 10 µM um 29,03%). Der Western Blot ist in Fig. 4 dargestellt.

### Beispiel 3

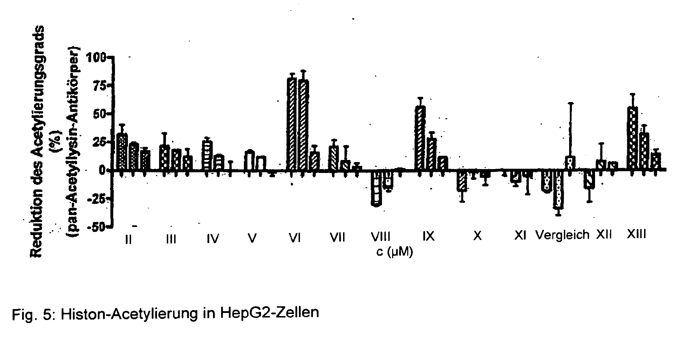

Für XIII wurde auch eine indirekte Beeinflussung des Acetylierungslevels gezeigt, worauf alle Zellen auch auf die Beeinflussung der Lysin-Acetylierung getestet wurden. Dieses Testsystem basiert ebenfalls auf den o.a. Bedingungen. Hier wurde jedoch ein pan-Anti-Acetyllysin-Antikörper verwendet. Die Ergebnisse sind in Fig. 5 dargestellt.

### Beispiel 4

Als besonders potent (Reduktion der Acetylierung an Lysinen in Core-1-Histonen > 50 % bei der Konzentration 25 µM) erwies sich hier XIII, das auch im in-vitro Testsystem bereits inhibitorisches Potential gegenüber SET7/9 gezeigt hatte. XII zeigte keinen Effekt auf die Acetylierung an Lysinen. Die Verbindungen VI und IX zeigten zusätzlich einen stark cytotoxischen Effekt.

### Beispiel 5

### Funktionaler Assay - Reduktion der Estrogen bzw. Androgen vermittelten Rezeptoraktivierung im transienten Transfektlonssystem

Die transienten Transfektionen wurden, um einen Einfluss der Testsubstanzen auf die Androgenrezeptor-vermittelte Genaktivierung zu ermitteln, in LnCap-Zellen durchgeführt. Diese exprimieren stabil den Androgenrezeptor (AR). Ein Einfluss der Testsubstanzen auf die Estrogenrezeptor α-vermittelte Genaktivierung wurde in HEK293-Zellen überprüft. Hier muss die DNA für den Estrogen-Rezeptor α (ER α) vor Testsubstanzzugabe in die Zellen hineinkloniert werden. Bei antiandrogener bzw. ―estrogener Wirkpotenz der Testsubstanzen ist die nachgelagerte Luziferaseaktivität vermindert bzw. nicht mehr vorhanden. Dies wird durch eine Reduzierung der Steroidrezeptorvermittelten Genaktivierung (Reduzierung um Faktor x im Vergleich zur Kontrolle) zum Ausdruck gebracht.

Besonders hohe antiandrogene Potenz zeigten die Verbindungen VI, IX, XII und XIII. Verglichen mit den übrigen Substanzen zeigten sie in der höchsten Konzentration eine mindestens 2-fach so hohe Reduzierung der androgenvermittelten Rezeptoraktivierung. Die Verbindungen VI und IX zeigten auch hier einen deutlichen cytotoxischen Effekt, welcher die eigentliche Wirkpotenz überlagert. Gleiches gilt für diese beiden Verbindungen bei der estrogenrezeptorvermittelten Aktivierung.

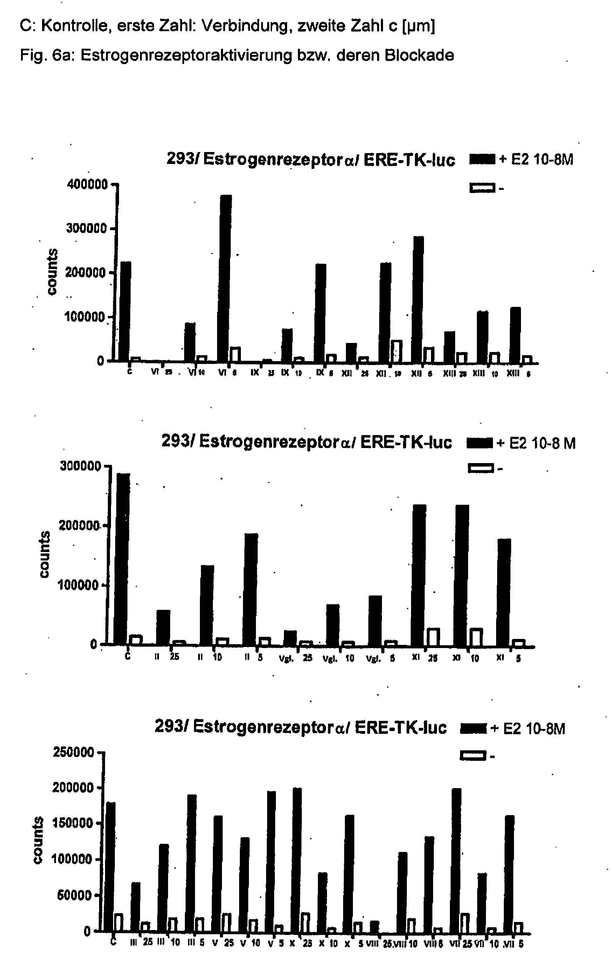

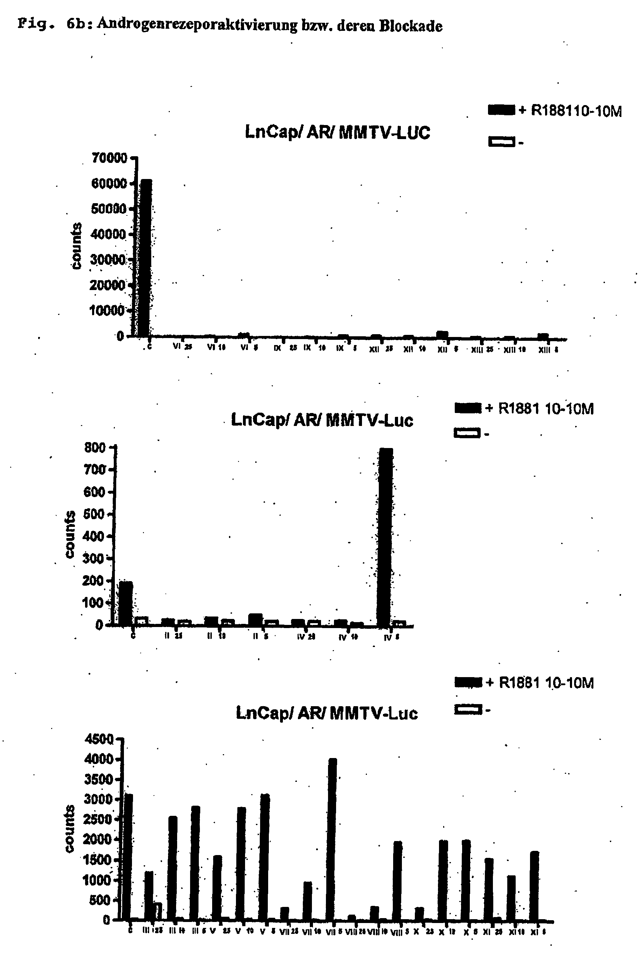

Insgesamt sind die neu identifizierten Verbindungen bei der estrogenrezeptovermittelten Aktivierung schwächer wirksam verglichen mit den Experimenten am Androgenrezeptor. XII und XIII zeigen auch hier das größte inhibitorische Potential, ohne jedoch einen cytotoxischen Effekt aufzuweisen (s. Fig. 6a und Fig 6b).

## Patentansprüche

1. Pharmazeutische Zusammensetzung, **dadurch gekennzeichnet, dass** sie eine Verbindung der allgemeinen Formel I 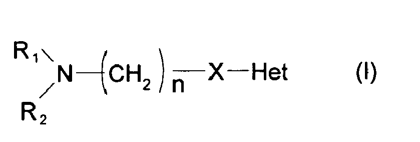 enthält, worin die Reste R₁ und R₂ gleich oder unterschiedlich sind und ausgewählt sind aus Wasserstoff, gerad- oder verzweigtkettiges C₁₋₅-Alkyl, oder 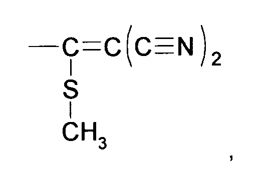 n eine ganze Zahl zwischen 3 und 5 ist,
X ein Heteroatom ist, ausgewählt aus O, S, N-R₁ oder die Bedeutung -CH₂- hat und der Rest Het ein 5-, 6- oder 7-gliedriger aromatischer oder nicht aromatischer Ring ist, der 1 bis 5 Heteroatome, ausgewählt aus N, S und O aufweist, wobei der Ring gegebenenfalls substituiert und/oder mit einem weiteren Ring annelliert sein kann und ein substituierter oder nicht substituierter polycyclischer Ring sein kann sowie dessen pharmazeutisch annehmbare Salze.

2. Pharmazeutische Zusammensetzung nach Anspruch 1, **dadurch gekennzeichnet, dass** der Rest R₁ und der Rest R₂ gleich oder unterschiedlich sind und ausgewählt sind aus Wasserstoff, Methyl und/oder Ethyl.

3. Pharmazeutische Zusammensetzung nach Anspruch 1 oder 2, **dadurch gekennzeichnet, dass** der Rest Het ein gegebenenfalls substituierter heterocyclischer Ring mit der Formel IA 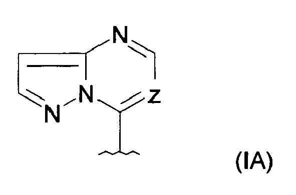 ist, in dem Z die Bedeutung CH oder N hat.

4. Pharmazeutische Zusammensetzung nach Anspruch 1 oder 2, **dadurch gekennzeichnet, dass** der Rest Het ausgewählt ist aus einer der nachfolgend aufgeführten Strukturen:

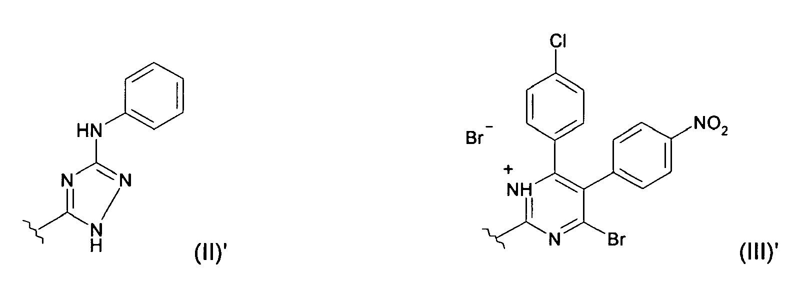

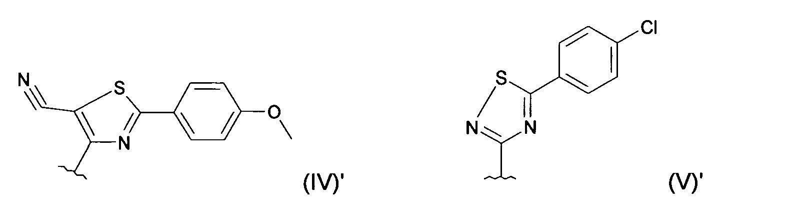

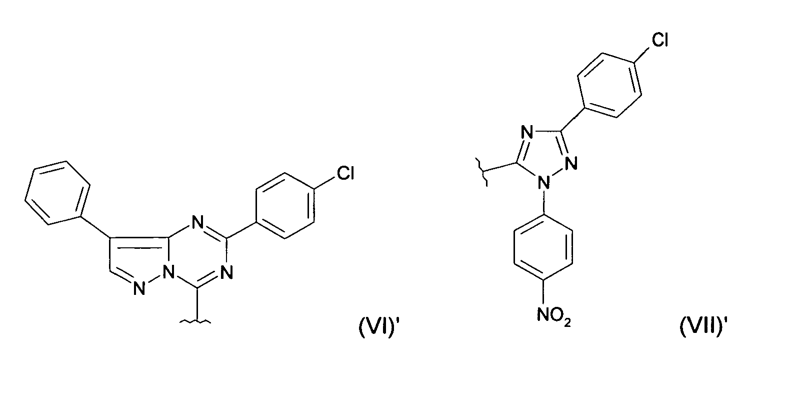

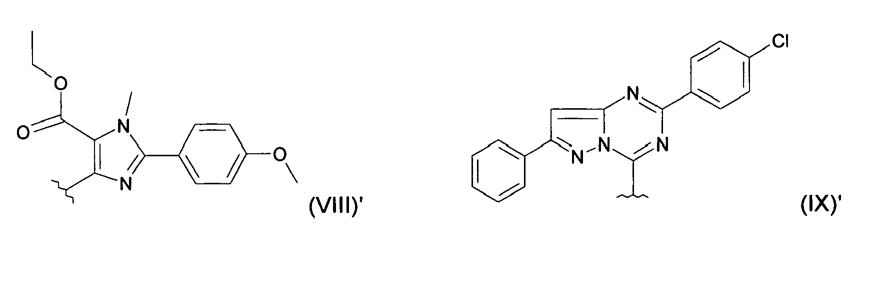

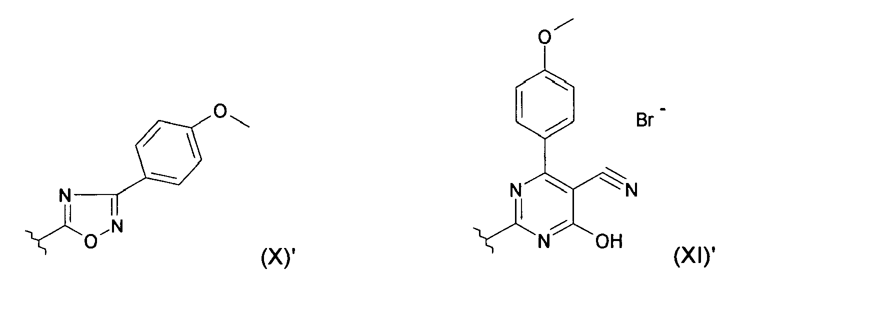

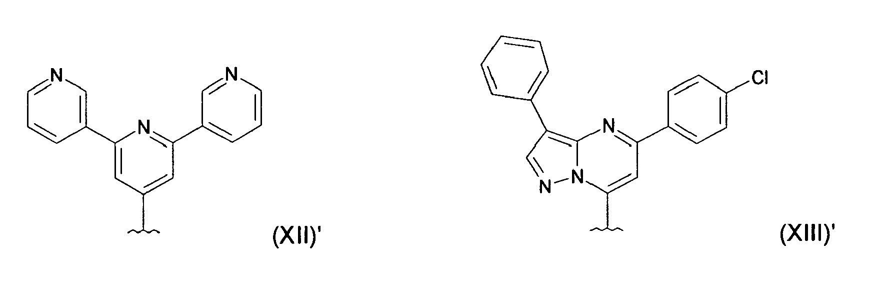

5. Pharmazeutische Zusammensetzung nach einem der Ansprüche 1-4 zur Verwendung als Antikrebsmittel.

6. Pharmazeutische Zusammensetzung nach Anspruch 5 als Mittel gegen einen Krebs oder Tumor, ausgewählt aus Blasenkarzinom, Brustkarzinom, Colonkarzinom, Nierenkarzinom, Leberkarzinom, Lungenkarzinom, kleinzelliges Lungenkarzinom, Speiseröhrenkarzinom, Gallenblasenkarzinom, Eierstockkarzinom, Pancreaskarzinom, Magenkarzinom, Zervixkarzinom, Prostatakarzinom, Karzinome der Haut, Leukämie, B-Zell-Lymphoma, T-Zell-Lymphoma, Hutchinson-Lymphoma, Non-Hutchinson-Lymphoma, Fibrosarkom, Neublastom, Melanom oder Karposi-Sarkom.

7. Pharmazeutische Zusammensetzung nach einem der Ansprüche 1-4, **dadurch gekennzeichnet, dass** diese in Kombination mit einer Anti-Androgen-wirksamen Verbindung eingesetzt wird.

8. Pharmazeutische Zusammensetzung nach Anspruch 7, **dadurch gekennzeichnet, dass** die Anti-Androgen-wirksame Verbindung ausgewählt ist aus der Gruppe umfassend Androgen-Rezeptorantagonisten und 5α-Reduktasehemmer.

9. Pharmazeutische Zusammensetzung nach Anspruch 8, **dadurch gekennzeichnet, dass** der Androgen-Rezeptorantagonist ausgewählt ist aus Flutamid, Bicalutamid, Cyproteron und Cyproteronacetat.

10. Pharmazeutische Zusammensetzung nach Anspruch 8, **dadurch gekennzeichnet, dass** der 5α-Reduktasehemmer ausgewählt ist aus Finasterid und Dutasterid.

11. Verwendung einer Verbindung der Formel (I) 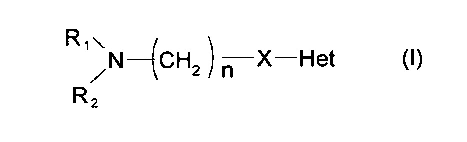 worin die Reste R₁ und R₂ gleich oder unterschiedlich sind und ausgewählt sind aus Wasserstoff, gerad- oder verzweigtkettiges C₁₋₅-Alkyl, oder 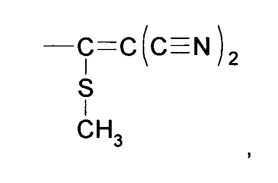 n eine ganze Zahl zwischen 3 und 5 ist,
X ein Heteroatom ist, ausgewählt aus O, S, N-R₁ oder die Bedeutung -CH₂- hat und der Rest Het ein 5-, 6- oder 7-gliedriger aromatischer oder nicht aromatischer Ring ist, der 1 bis 5 Heteroatome, ausgewählt aus N, S und O aufweist, wobei der Ring gegebenenfalls substituiert und/oder mit einem weiteren Ring annelliert sein kann und ein substituierter oder nicht substituierter polycyclischer Ring sein kann sowie dessen pharmazeutisch annehmbare Salze, zur Herstellung eines Medikaments zur Behandlung oder Linderung einer Krebserkrankung.

12. Verwendung nach Anspruch 11, **dadurch gekennzeichnet, dass** die Krebs- und/oder Tumorerkrankung ausgewählt ist aus folgenden Tumorerkrankungen: Blasenkarzinom, Brustkarzinom, Colonkarzinom, Nierenkarzinom, Leberkarzinom, Lungenkarzinom, kleinzelliges Lungenkarzinom, Speiseröhrenkarzinom, Gallenblasenkarzinom, Eierstockkarzinom, Pancreaskarzinom, Magenkarzinom, Zervixkarzinom, Prostatakarzinom, Karzinome der Haut, Leukämie, B-Zell-Lymphoma, T-Zell-Lymphoma, Hutchinson-Lymphoman, Non-Hutchinson-Lymphoma, Fibrosarkom, Neublastom, Melanom oder Karposi-Sarkom.
